# EUROPÄISCHE PATENTSCHRIFT

(11) **EP 1 066 064 B1**
(45) Veröffentlichungstag und Bekanntmachung des Hinweises auf die Patenterteilung: **28.04.2004**
(21) Anmeldenummer: 99914546.9
(22) Anmeldetag: 26.03.1999
(51) Int. Cl.: A61L 15/12, A61F 13/04

(54) **SYNTHETISCHER STEIFVERBAND**
SYNTHETIC RIGID BANDAGE
BANDAGE SYNTHETIQUE RIGIDE

(30) Priorität: 02.04.1998 DE 19814826; 11.09.1998 DE 19841561
(43) Veröffentlichungstag der Anmeldung: 10.01.2001
(73) Patentinhaber: Lohmann GmbH & Co. KG, 56567 Neuwied (DE)
(72) Erfinder: BRANDHOFF, Stefan, D-56626 Andernach (DE)
(74) Vertreter: Flaccus, Rolf-Dieter, Dr.
(86) Internationale Anmeldenummer: PCT/EP1999/002077
(87) Internationale Veröffentlichungsnummer: WO 1999/051281

(56) Entgegenhaltungen:
- EP-A1- 0 393 003
- WO-A1-95/19751
- DE-A1- 2 357 931
- US-A- 3 656 475
- US-A- 4 326 509
- US-A- 4 899 738
- US-A- 4 968 542
- US-A- 5 027 803

## Beschreibung

Die Erfindung betrifft einen synthetischen Steifverband, insbesondere zum Schienen und/oder Stützen eines erkrankten oder verletzten Gliedes am menschlichen oder tierischen Bewegungsapparat, umfassend einen durch eine oder mehrere mit Kunstharz getränkte Lagen von Längsstreifen eines textilen Flächengebildes ausgebildeten, mit einem Polster auf der einen Seite und einer wasserdampfdurchlässigen, längs- und/oder querelastischen Folienabdeckung auf der anderen Seite abgedeckten und in einer feuchtigkeitsdichten Verpakkung aufzubewahrenden, härtbaren Kern, der im applikationsbereiten Zustand modellierbar ist.

Beim Stand der Technik sind in einer Vielzahl von Dokumenten orthopädische Schienen oder Stützen beschrieben, deren Material sowie Mittel zur Herstellung denjenigen gemäß Oberbegriff von Anspruch 1 des Anmeldungsgegenstandes prinzipiell weitgehend ähneln.
Als Ausgangsmaterial werden zumeist textile Substrate aus Natur- oder Kunststoff- und vielfach Glasfasern verwendet, getränkt mit einem härtbaren Kunststoffharz und bis zur Verwendung in einer Folienhülle, vor Zutritt eines Härtemediums geschützt, zumeist in Form einer Rolle aufbewahrt. Als Härter sind unter anderen vorgesehen:
a) UV- bzw. Röntgenstrahlen; z.B. im Zusammenwirken mit Polyurethan und einem Photoinitiator gemäß US 3 656 475.
b) Wärmeeinwirkung; z.B. wird eine orthopädische Bandage aus dickem textilem Material mit einer thermoplastischen Komposition, enthaltend 60-80 Gew.% saturierten linearen Polyester mit 20-40 Gew.% Harz von niedrigem Kristallisationspunkt und Erweichungstemperatur bei 45 °C gehärtet gemäß US 4 326 509.
c) Luftfeuchtigkeit bzw. Wasser; z.B. wird ein textiles Substrat, imprägniert mit Harz aus der Gruppe wasserhärtbarer Isocyanate in einer porösen Hülle aufbewahrt und zum Härten mit Wasser getränkt, gemäß DE-PS 23 57 931.
d) Halbsteife, nachgiebige Stützen für beschädigte oder erkrankte Glieder sind aus US 4 968 542 bekannt und werden ebenfalls durch eine Reaktion zwischen Harz und Härter gehärtet.

Bei der Vorratshaltung harzimprägnierter textiler Substrate hat sich aus vielfacher negativer Erfahrung als Nachteil herausgestellt, daß bei gängigen Systemen der vorbeschriebenen Art mit harzgetränkten Kernen nach einer nicht vorherbestimmbaren Lagerzeit ein Harzdurchschlag durch die Abdeckung erfolgt. Hierdurch kommt es zum Hautkontakt mit den Händen des Anwenders und damit zu einer möglichen Gefährdung, da ohne entsprechende Schutzeinrichtung (Handschuhe) gearbeitet werden soll. Darüber hinaus wird das Produkt unbrauchbar.

Der Erfindung liegt die Aufgabe zugrunde, einen synthetischen Steifverband der im Oberbegriff von Anspruch 1 genannten Art derart zu verbessern, daß bei einer Vorratshaltung auch über einen längeren Zeitraum von harzimprägnierten textilen Flächengebilden oder Vliesstoff der Durchtritt von Harz durch die Abdeckung unter Verwendung unkomplizierter und preisgünstiger Mittel sicher verhindert wird.

Zur Lösung der Aufgabe wird bei einem synthetischen Steifverband der eingangs genannten Art mit der Erfindung vorgeschlagen, daß ein Abstandshalter zwischen der Folienabdekkung und dem harzgetränkten Kern eingefügt wird, der mit der Folie als Außenschicht durch eine segmentierte, wasserdampfdurchlässige Haftklebebeschichtung verbunden wird.

Durch die Aufbringung z.B. eines hydrophoben, luftdurchlässigen und längs- und/oder querelastischen Vlieses, einer Watte, eines Schaumstoffes oder eines textilen Flächengebildes auf der Innenwand der Abdeckfolie wird ein nachteiliger Kontakt zwischen Harz und Folie verhindert, so daß ein Harzdurchschlag mit seinen nachteiligen Folgen vollständig vermieden wird.

Der Abstandshalter saugt das Harz nicht aus den Lagen des Kernes heraus und die segmentierte Haftklebebeschichtung zwischen Abstandshalter und Folie führt zu einer optimalen ganzheitlichen Lösung, die einerseits die wasserdampfdurchlässige Eigenschaft der Folie nicht verringert und andererseits mit vernachlässigbar geringen Kosten durchführbar ist. Dabei bleibt auch das auf diese Weise gebildete Laminat längs- und querelastisch, so daß sich der Steifverband faltenfrei an ein Körperteil, beispielsweise einen Arm oder ein Bein, anlegen läßt, darüber hinaus in einer angemessenen Zeit trocknet.

Weitere Ausgestaltungen der Erfindung sind entsprechend den Unteransprüchen vorgesehen.
Eine Ausgestaltung des Steifverbandes sieht vor, daß die den Kern bildenden Lagen von Längsstreifen mit Polyurethanharz getränkt sind.
Dieses Harz eignet sich in besonderer Weise zur Aushärtung mittels Luftfeuchtigkeit oder Wasser.

Es kann von der Maßnahme Gebrauch gemacht sein, daß das Vlies zu 100 % aus Polyesterfasern besteht.
Weiterhin ist mit der Erfindung vorgesehen, daß die Lage Vlies mit der Abdeckfolie ein Laminat bildet, das längs- und querelastisch und wasserdampfdurchlässig ist. Schließlich sieht eine Ausgestaltung vor, daß die Schicht der hydrophoben Synthetikwatte eine Dicke zwischen 1 und 8 mm, bevorzugt zwischen 1,5 und 5 mm und besonders bevorzugt von 2,5 mm aufweist.

Die Anwendung des synthetischen Steifverbandes nach der Erfindung ist unkompliziert und zeitsparend, weil die übliche Polsterung eines einfachen Steifverbandes entbehrt werden kann. Die Handhabung ist einfach, Handschuhe werden nicht benötigt, der sogenannte Gipsraum bleibt sauber. Je nach Indikation wird die Breite und Länge des Steifverbandes gewählt und die erforderliche Länge aus der Verpakkung entnommen.
Die Schnittstelle wird sofort sorgfältig verschlossen, um ein Aushärten durch Eintritt von Luftfeuchtigkeit zu vermeiden. Dazu wird bevorzugt das Materialende einige Zentimeter nach innen umgelegt und feuchtigkeitsdicht verschlossen. Sodann wird der abgetrennte Steifverband in auf ca. 20-30 °C temperiertes Wasser getaucht, anschließend gefaltet und überschüssiges Wasser ausgedrückt. Sodann wird durch leichtes Strecken das Polster über die rauhen Glasfaserschnittkanten gezogen.
Zur Applikation wird das formbare Material mit einer elastischen Binde an einem Ende fixiert, anschließend mit der Hand an das zu stützende Körperteil anmodelliert und sodann möglichst ohne Bewegung abgewartet, bis der Steifverband in 3-5 min abgebunden hat.

Die Erfindung ist unkompliziert und preisgünstig und verhindert den unerwünschten Kontakt zwischen Harz und Folie, so daß der Harzdurchschlag mit seinen Nachteilen vermieden wird.
Insofern löst die Erfindung in optimaler Weise die eingangs gestellte Aufgabe.

## Patentansprüche

1. Synthetischer Steifverband, insbesondere zum Schienen und/oder Stützen eines erkrankten oder verletzten Gliedes am menschlichen oder tierischen Bewegungsapparat, umfassend einen durch eine oder mehrere kunstharzgetränkte Lagen von Längsstreifen eines textilen Flächengebildes ausgebildeten, mit einem Polster auf der einen Seite und einer wasserdampfdurchlässigen, längs- und/oder querelastischen Folienabdeckung auf der anderen Seite abgedeckten und in einer feuchtigkeitsdichten Verpackung aufzubewahrenden, härtbaren Kern, der im applikationsbereiten Zustand modellierbar ist, **dadurch gekennzeichnet, daß** die Folienabdeckung durch einen Abstandshalter aus einem hydrophoben, luftdurchlässigen, längs- und/oder querelastischen Vlies von den harzgetränkten Lagen getrennt ist.

2. Steifverband nach Anspruch 1, **dadurch gekennzeichnet, daß** das den Kern bildende, textile Flächengebilde mit Polyurethanharz getränkt ist.

3. Steifverband nach Anspruch 1 oder 2, **dadurch gekennzeichnet, daß** der Abstandshalter durch eine wasserdampfdurchlässige Haftklebebeschichtung mit der Abdeckfolie verbunden ist.

4. Steifverband nach Anspruch 3, **dadurch gekennzeichnet, daß** die Haftklebebeschichtung segmentiert ist.

5. Steifverband nach einem oder mehreren der Ansprüche 1 bis 4, **dadurch gekennzeichnet, daß** der Abstandshalter mit der äußeren Kunststofffolie ein Laminat bildet, das längs- und querelastisch ist.

6. Steifverband nach einem oder mehreren der Ansprüche 1 bis 5, **dadurch gekennzeichnet, daß** der Abstadshalter eine Dicke zwischen 1 und 8 mm, bevorzugt zwischen 1,5 und 5 mm und besonders bevorzugt von 2,5 mm aufweist.

## Claims

1. Synthetic rigid bandage especially for splinting and/or supporting a diseased or injured limb of the human or animal apparatus of locomotion, comprising a curable core which is formed by one or more synthetic resin-impregnated layers of longitudinal strips of a textile fabric, is covered at the one side thereof with a pad and on the other side thereof with a water vapour-pervious, longitudinally and/or transversally elastic film or sheet cover, and is to be stored in a moisture-proof package, said core being mouldable in its ready-for-application state, **characterized in that** the said film or sheet cover is separated from the said resin-impregnated layers by means of a spacer made up of a hydrophobic, air-permeable, longitudinally and/or transversally elastic nonwoven.

2. Rigid bandage according to Claim 1, **characterized in that** the textile fabric forming the core is impregnated with polyurethane resin.

3. Rigid bandage according to Claim 1 or 2, **characterized in that** the spacer is connected with the covering film or sheet by means of a water vapour-permeable pressure-sensitive adhesive coating.

4. Rigid bandage according to Claim 3, **characterized in that** the pressure-sensitive adhesive coating is segmental.

5. Rigid bandage according to one or more of Claims 1 to 4, **characterized in that** the spacer forms a laminate with the outer plastics film, which laminate is longitudinally and transversally elastic.

6. Rigid bandage according to one or more of Claims 1 to 5, **characterized in that** the spacer has a thickness of between 1 and 8 mm, preferably between 1.5 and 5 mm, and especially preferred 2.5 mm.

## Revendications

1. Bandage synthétique rigide, destiné en particulier à servir d'attelle et/ou de soutien à un membre malade ou blessé de l'appareil locomoteur humain ou animal, comprenant un noyau durcissable recouvert et à conserver dans un emballage étanche à l'humidité, formé d'une ou dé plusieurs couches imprégnées de résine synthétique de bandes longitudinales d'une structure textile superficielle comportant un matelas d'un côté et un recouvrement à feuille perméable à la vapeur d'eau, élastique dans le sens longitudinal et dans le sens transversal de l'autre côté, lequel noyau peut être modelé à l'état prêt à être appliqué, **caractérisé en ce que** le recouvrement à feuille est séparé des couches imprégnées de résine par un intercalaire constitué d'une toile hydrophobe, perméable à l'air et élastique dans le sens longitudinal et dans le sens transversal.

2. Bandage rigide selon la revendication 1, **caractérisé en ce que** la structure textile superficielle formant le noyau est imprégnée de résine polyuréthane.

3. Bandage rigide selon la revendication 1 ou 2, **caractérisé en ce que** l'intercalaire est relié à la feuille de recouvrement par un revêtement auto-adhésif perméable à la vapeur d'eau.

4. Bandage rigide selon la revendication 3, **caractérisé en ce que** le revêtement auto-adhésif est segmenté.

5. Bandage rigide selon une ou plusieurs des revendications 1 à 4 **caractérisé en ce que** l'intercalaire forme un stratifié avec la feuille synthétique extérieure, lequel est élastique dans le sens longitudinal et dans le sens transversal.

6. Bandage rigide selon une ou plusieurs des revendications 1 à 5, **caractérisé en ce que** l'intercalaire présente une épaisseur comprise entre 1 et 8 mm, de préférence entre 1,5 et 5 mm et particulièrement de préférence de 2,5 mm.
